# EUROPÄISCHE PATENTSCHRIFT

(11) **EP 0 943 318 B1**
(45) Veröffentlichungstag und Bekanntmachung des Hinweises auf die Patenterteilung: **02.04.2003**
(21) Anmeldenummer: 99101160.2
(22) Anmeldetag: 22.01.1999
(51) Int. Cl.: A61K 7/13

(54) **Haarfärbemittel**
Hair-dye
Teinture pour cheveux

(30) Priorität: 21.02.1998 DE 19807508
(43) Veröffentlichungstag der Anmeldung: 22.09.1999
(73) Patentinhaber: KPSS-Kao Professional Salon Services GmbH, 64297 Darmstadt (DE)
(72) Erfinder: Kufner, Frank, 64297 Darmstadt (DE); Lorenz, Heribert, 64401 Gross-Bieberau (DE)

(56) Entgegenhaltungen:
- WO-A-94/01076
- WO-A-97/13498
- DE-U- 9 101 245
- DE-U- 9 107 663

## Beschreibung

Die vorliegende Erfindung betrifft Haarfärbemittel auf Basis einer wäßrigen Emulsion, enthaltend mindestens ein Oxidationsfarbstoff-Vorprodukt, insbesondere ein solches in Form einer wäßrigen Emulsion, mit verbesserter Viskositätsstabilität.

Permanente Haarfärbemittel auf Basis von Oxidationsfarbstoffen erfreuen sich einer weiten Verbreitung. Ihre Anwendung erfolgt in der Regel dergestalt, daß eine flüssige, zumeist als wäßrige Emulsion vorliegende, mindestens ein Oxidationsfarbstoff-Vorprodukt, im allgemeinen mindestens eine Entwickler- und mindestens eine Kupplersubstanz, enthaltende Zusammensetzung unmittelbar vor der Anwendung mit einer Peroxid enthaltenden Zusammensetzung vermischt, und die Mischung auf das Haar aufgebracht wird.

Dabei ist die Einhaltung gewisser Viskositätsverhältnisse zweckmäßig. Es hat sich jedoch gezeigt, daß diese Emulsionen häufig thixotrope Eigenschaften aufweisen, d.h., bei der Lagerung in der Dose oder Tube ihre Viskosität erhöhen, was dann bei der Applikation und der Vermischung mit der Peroxid-Zusammensetzung Probleme bereitet.

Die Erfindung geht daher von der Aufgabenstellung aus, diese Probleme zu vermeiden und Haarfärbeemulsionen mit stabiler Viskosität zur Verfügung zu stellen.

Erfindungsgemäß wurde festgestellt, daß wäßrige Haarfärbe-Emulsionen, die mindestens ein Oxidationsfarbstoff-Vorprodukt enthalten, dann viskositätsstabil sind, wenn sie ein Gemisch aus 0,5 bis 5 Gew.-% mindestens eines Ethandiol- und/oder 1,2-Propandiol-C₁₂-C₂₀-Fettsäurediesters und 0,5 bis 5 Gew.-% mindestens eines C₁₀-C₂₀-Alkylamidobetains, jeweils berechnet auf die Gesamtzusammensetzung der Emulsion, enthalten.

Gemäß einer bevorzugten Ausführungsform der Erfindung werden als Ethandiol- bzw. 1,2-Propandiolfettsäurediester die Stearate eingesetzt, doch ist auch die Verwendung der Dilaurate, Dimyristate, Dipalmitate oder Dioleate sowie von Mischestern oder Estergemischen möglich.
Die bevorzugte Menge liegt bei etwa 1 bis 2,5, insbesondere bei etwa 2 Gew.-%, berechnet auf die Gesamtzusammensetzung.

Als C₁₀-C₂₀-Alkylamidopropylbetain wird vorzugsweise Kokosmidopropylbetain in einer Menge von etwa 1 bis 2,5, insbesondere etwa 2 Gew.-%, berechnet auf die Gesamtzusammensetzung, eingesetzt.

Das bevorzugte Gewichtsverhältnis beider Komponenten liegt bei 1:3 bis 3:1, insbesondere etwa 1:1.

Gemäß einer weiteren bevorzugten Ausführungsform der Erfindung sind in der Haarfärbe-Emulsion noch 0,5 bis 5, vorzugsweise etwa 1 bis 2,5, insbesondere etwa 2 Gew.-%, berechnet auf die Gesamtzusammensetzung, mindestens eines C₆-C₁₈-Fettsäuretriglycerids vorhanden.
Als solches wird Caprinsäuretriglycerid bevorzugt, jedoch sind grundsätzlich auch andere Triglyceride verwendbar, z.B., jene von Caprylsäure, Laurinsäure, Myristinsäure oder Palmitinsäure sowie solche von Mischsäuren, z.B. Kokosölfettsäuren.

Die sonstigen möglichen Bestandteile der erfindungsgemäßen Haarfärbemittel sind an sich bekannt.

Diese können üblicherweise weitere Emulgatoren und Tenside, Lösungsvermittler, Verdickungsmittel, haarkonditionierende Substanzen wie Eiweißhydrolysate und synthetische Polymere, Stabilisatoren, Verdünnungsmittel, etc. enthalten.

Ausführliche Angaben über die Zusammensetzung und Herstellung von Haarfärbemitteln finden sich beispielsweise in der Monographie von K. Schrader, Grundlagen und Rezepturen der Kosmetika (1989, 2. Auflage, Dr. A. Hüthig Verlag), S. 782-815, auf die hier ausdrücklich Bezug genommen wird.

Die Anwendung der Oxidationsfarbstoff-Vorprodukte enthaltenden Haarfärbemittel erfolgt durch Vermischen mit Peroxiden, insbesondere Wasserstoffperoxidlösung oder -lotion, unmittelbar vor der Applikation auf das Haar.

Die auf das Haar aufzubringende, gebrauchsfertige Färbemischung weist dabei vorzugsweise einen alkalischen pH-Wert im Bereich von 7,5 bis 9,5 auf, jedoch sind auch neutrale und schwach saure Endprodukte geeignet, z.B. im Bereich von etwa 5,5 bis etwa 7,5.

Aus dem älteren deutschen Patent Nr. 197 01 422.4 sind Haarfärbemittel in einer Grundlage bekannt, die 0,25 bis 5 Gew.-% mindestens eines ethoxylierten C₁₀-C₂₀-Fettsäurealkanolamids enthalten.
Die Mitverwendung solcher ethoxylierten C₁₀-C₂₀-Fettsäurealkanolamide in der erfindungsgemäßen Zusammensetzung ist vom Schutzumfang ausdrücklich ausgenommen.

Im folgenden wird anhand der Ausführungsbeispiele die Wirkung der erfindungsgemäßen Zusammensetzungen beschrieben.

| | A | B | C | D | E |
|---|---|---|---|---|---|
| Cetylstearylalkohol | 10,00 | 8,00 | 8,00 | 8,00 | 8,00 |
| Stearinsäuremonoethanolamid | 2,00 | 2,00 | 2,00 | 2,00 | 2,00 |
| Kokossäuremonoethanolamid | 2,00 | 2,00 | 2,00 | 2,00 | 2,00 |
| Ölsäure | 1,00 | 1,00 | 1,00 | 1,00 | 1,00 |
| Ethanoldioldistearat | - | 2,00 | - | 1,00 | 2,00 |
| Kokoamidopropylbetain | - | 2,00 | 2,00 | 1,00 | - |
| Caprinsäuretriglycerid | - | - | - | 2,00 | - |
| Hydroxyethlcellulose | 0,50 | 0,50 | 0,50 | 0,50 | 0,50 |
| Natriumlaurylsulfat | 0,50 | 0,50 | 0,50 | 0,50 | 0,50 |
| Tetranatrium-EDTA | 0,20 | 0,20 | 0,20 | 0,20 | 0,20 |
| Ammoniumchlorid | 0,25 | 0,25 | 0,25 | 0,25 | 0,25 |
| Natriumsulfit | 0,50 | 0,50 | 0,50 | 0,50 | 0,50 |
| Natriumhydroxid | 0,48 | 0,48 | 0,48 | 0,48 | 0,48 |
| Mangandioxid | 0,12 | 0,12 | 0,12 | 0,12 | 0,12 |
| Proteinhydrolysat | 1,00 | 1,00 | 1,00 | 1,00 | 1,00 |
| Parfum | 0,20 | 0,20 | 0,20 | 0,20 | 0,20 |
| 2,5-Diaminotoluolsulfat | 0,22 | 0,22 | 0,22 | 0,22 | 0,22 |
| 4-Aminophenol | 0,10 | 0,10 | 0,10 | 0,10 | 0,10 |
| 2-Methylresorcin | 0,05 | 0,05 | 0,05 | 0,05 | 0,05 |
| 3-Aminophenol | 0,05 | 0,05 | 0,05 | 0,05 | 0,05 |
| 2,5,6-Triamino-4-hydroxypyrimidinsulfat | 0,10 | 0,10 | 0,10 | 0,10 | 0,10 |
| Wasser ad | 100,00 | 100,00 | 100,00 | 100,00 | 100,00 Gew.-% |

Viskiositätsmessungen zeigten, daß sich die Zusammensetzung D nach 1, 2, 3 und 4 Monaten am stabilsten erwies, gefolgt von der Zusammensetzung B.

Die Viskosität der Zusammensetzungen C und E stieg etwa im gleichen Umfang an; der Viskositätswert der Zusammensetzung A, einer handelsüblichen Zusammensetzung, verdoppelte sich nach 4 Monaten.

## Patentansprüche

1. Haarfärbemittel auf Basis einer wäßrigen Emulsion, enthaltend mindestens ein Oxidationsfarbstoff-Vorprodukt, **dadurch gekennzeichnet, daß** die Emulsion ein Gemisch aus
a) 0,5 bis 5 Gew.-% mindestens eines Ethandiol- und/oder 1,2-Propandiol-C₁₂-C₂₀-Fettsäurediesters, und
b) 0,5 bis 5 Gew.-% mindestens eines C₁₀-C₂₀-Alkylamidopropylbetains, jeweils berechnet auf die Gesamtzusammensetzung des Mittels, enthält, und das Mittel frei von ethoxylierten C₁₀-C₂₀-Fettsäurealkanolamiden ist.

2. Harrfärbemittel nach Anspruch 1, **dadurch gekennzeichnet, daß** es zusätzlich 0,5 bis 5 Gew.-%, berechnet auf die Gesamtzusammensetzung, mindestens eines C₆-C₁₈-Fettsäuretriglycerids enthält.

3. Haarfärbemittel nach Anspruch 1 und/oder 2, **dadurch gekennzeichnet, daß** es die Bestandteile a) und b) in einem Gewichtsverhältnis von 1 zu 3 bis 3 zu 1 enthält.

4. Haarfärbemittel nach einem oder mehreren der Ansprüche 1 bis 3, **dadurch gekennzeichnet, daß** es als Bestandteil a) Ethandioldistearat enthält.

5. Haarfärbemittel nach einem oder mehreren der Ansprüche 1 bis 4, **dadurch gekennzeichnet, daß** es als Bestandteil b) Kokosamidopropylbetain enthält.

6. Haarfärbemittel nach einem oder mehreren der Ansprüche 2 bis 5, **dadurch gekennzeichnet, daß** es als Fettsäuretriglycerid Caprinsäuretriglycerid enthält.

## Claims

1. Hair dyeing composition on the basis of an aqueous emulsion, comprising at least one oxidation dyestuff precursor, wherein the emulsion comprises a mixture of
a) 0.5% to 5% by weight of at least one ethanediol- and/or 1,2-propanediol-C₁₂-C₂₀-fatty acid diester, and
b) 0.5% to 5% by weight of at least one C₁₀-C₂₀-alkyl amidopropyl betaine, each calculated to the total composition, and the composition being free from ethoxylated C₁₀-C₂₀-fatty acid alkanolamides.

2. Hair dyeing composition according to claim 1, additionally comprising 0.5% to 5% by weight, calculated to the total composition, of at least one C₆-C₁₈-fatty acid triglyceride.

3. Hair dyeing composition according to claims 1 and/or 2, wherein the components a) and b) have a weight proportion of 1 to 3 to 3 to 1.

4. Hair dyeing composition according to one or more of claims 1 to 3, wherein component a) consists of ethanediol distearate.

5. Hair dyeing composition according to one or more of claims 1 to 4, wherein component b) consist of cocoamidopropyl betaine.

6. Hair dyeing composition according to one or more of claims 2 to 5, comprising capric acid triglyceride as fatty acid triglyceride.

## Revendications

1. Colorant pour cheveux à base d'une émulsion aqueuse, contenant au moins un progéniteur de colorant d'oxydation, **caractérisé en ce que** l'émulsion contient un mélange constitué de
a) au moins un diester de l'acide gras en C₁₂ à C₂₀ et du 1,2-propanediol et/ou un diester de l'acide gras en C₁₂ à C₂₀ et de l'éthanediol, à raison de 0,5% à 5% en poids, et
b) au moins une bétaïne d'(alkyle en C₁₀ à C₂₀)-amidopropyle, à raison de 0,5% à 5% en poids,
à chaque fois calculé par rapport à la composition totale du colorant, et **caractérisé en ce que** le colorant est exempt d'alcanolamides d'(acide gras en C₁₀ à C₂₀) éthoxylés.

2. Colorant pour cheveux selon la revendication 1, **caractérisé en ce qu'**il contient, en plus, au moins un triglycéride d'(acide gras en C₆ à C₁₈), à raison de 0,5% à 5% en poids, calculé par rapport à la composition totale.

3. Colorant pour cheveux selon la revendication 1 et/ou 2, **caractérisé en ce qu'**il contient les composants a) et b) dans un rapport pondéral compris entre 1 à 3 et 3 à 1.

4. Colorant pour cheveux selon une ou plusieurs des revendications 1 à 3, **caractérisé en ce qu'**il contient, en tant que composant a), le distéarate d'éthanediol.

5. Colorant pour cheveux selon une ou plusieurs des revendications 1 à 4, **caractérisé en ce qu'**il contient, en tant que composant b), la bétaïne de coco-amidopropyle.

6. Colorant pour cheveux selon une ou plusieurs des revendications 2 à 5, **caractérisé en ce qu'**il contient, en tant que triglycéride d'acide gras, le triglycéride d'acide caprique.
